# EUROPEAN PATENT SPECIFICATION

(11) **EP 2 603 226 B1**
(45) Date of publication and mention of the grant of the patent: **07.04.2021**
(21) Application number: 11817033.1
(22) Date of filing: 11.08.2011
(51) Int. Cl.: A61K 35/12, A61K 38/18, A61K 38/22, A61P 3/10, C12N 5/00

(54) **TREATMENT OF DIABETES WITH PANCREATIC ENDOCRINE PRECURSOR CELLS**
BEHANDLUNG VON DIABETES MIT ENDOKRINEN PANKREAS-VORLÄUFERZELLEN
TRAITEMENT DU DIABÈTE AU MOYEN DE CELLULES PRÉCURSEURS ENDOCRINES PANCRÉATIQUES

(30) Priority: 12.08.2010 US 373109 P
(43) Date of publication of application: 19.06.2013
(73) Proprietor: Janssen Biotech, Inc., Horsham, PA 19044 (US)
(72) Inventor: XU, Jean, Skillman NJ 08558 (US)
(74) Representative: Vossius & Partner Patentanwälte Rechtsanwälte mbB
(86) International application number: PCT/US2011/047410
(87) International publication number: WO 2012/021698

(56) References cited:
- WO-A1-01/23528
- WO-A1-2009/018453
- WO-A2-2010/057039
- US-A1- 2004 121 460
- US-A1- 2007 154 981
- Jayne L Foster ET AL: "Differentiation of transplanted microencapsulated fetal pancreatic cells", Transplantation, 15 June 2007 (2007-06-15), pages 1440-1448, XP055091824, United States DOI: 10.1097/01.tp.0000264555.46417.7d Retrieved from the Internet: URL:http://www.ncbi.nlm.nih.gov/pubmed/175 65317
- A. V. MATVEYENKO ET AL: "Inconsistent formation and nonfunction of insulin-positive cells from pancreatic endoderm derived from human embryonic stem cells in athymic nude rats", AJP: ENDOCRINOLOGY AND METABOLISM, vol. 299, no. 5, 29 June 2010 (2010-06-29) , pages E713-E720, XP055091836, ISSN: 0193-1849, DOI: 10.1152/ajpendo.00279.2010
- LEE SEUNG-HEE ET AL: "Human beta-cell Precursors Mature Into Functional Insulin-producing Cells in an Immunoisolation Device: Implications for Diabetes Cell Therapies", TRANSPLANTATION, WILLIAMS AND WILKINS, BALTIMORE US, vol. 87, no. 7, 15 April 2009 (2009-04-15) , pages 983-991, XP009174839, ISSN: 0041-1337
- RAMIYA V K ET AL: "REVERSAL OF INSULIN-DEPENDENT DIABETES USING ISLETS GENERATED IN VITRO FROM PANCREATIC STEM CELLS", NATURE MEDICINE, NATURE PUBLISHING GROUP, NEW YORK, NY, US, vol. 6, no. 3, 1 March 2000 (2000-03-01), pages 278-282, XP002931497, ISSN: 1078-8956, DOI: 10.1038/73128
- DONG-QI T. ET AL.: 'Reprogramming liver-stem WB cells into functional insulin-producing cells by persistent expression of Pdxl- and Pdxl-VP16 mediated by lentiviral vectors' LABORATORY INVESTIGATION vol. 86, no. 1, 2006, pages 83 - 93, XP055076115
- MOVASSAT J. ET AL.: 'Keratinocyte growth factor and beta-cell differentiation in human fetal pancreatic endocrine precursor cells' DIABETOLOGIA vol. 46, no. 6, 2003, pages 822 - 829, XP055076116
- REISNER Y.: 'Growing organs for transplantation from embryonic precursor tissues' IMMUNOL. RES. vol. 38, no. 1/3, 2007, pages 261 - 273, XP055076117

## Description

### FIELD OF THE INVENTION

The present invention provides a population of encapsulated pancreatic endocrine precursor cells for use in a method of lowering blood glucose levels in an animal, as defined in the claims.

### BACKGROUND

Advances in cell-replacement therapy for Type I diabetes mellitus and a shortage of transplantable islets of Langerhans have focused interest on developing sources of insulin-producing cells, or β cells, appropriate for engraftment. One approach is the generation of functional β cells from pluripotent stem cells, such as, for example, embryonic stem cells.

In vertebrate embryonic development, a pluripotent cell gives rise to a group of cells comprising three germ layers (ectoderm, mesoderm, and endoderm) in a process known as gastrulation. Tissues such as, for example, thyroid, thymus, pancreas, gut, and liver, will develop from the endoderm, via an intermediate stage. The intermediate stage in this process is the formation of definitive endoderm. Definitive endoderm cells express a number of markers, such as, for example, HNF3 beta, GATA4, MIXL1, CXCR4 and SOX17.

Formation of the pancreas arises from the differentiation of definitive endoderm into pancreatic endoderm. Cells of the pancreatic endoderm express the pancreatic-duodenal homeobox gene, PDX1. In the absence of PDX1, the pancreas fails to develop beyond the formation of ventral and dorsal buds. Thus, PDX1 expression marks a critical step in pancreatic organogenesis. The mature pancreas contains, among other cell types, exocrine tissue and endocrine tissue. Exocrine and endocrine tissues arise from the differentiation of pancreatic endoderm.

Cells bearing the features of islet cells have reportedly been derived from embryonic cells of the mouse. For example, Lumelsky et al. (Science 292:1389, 2001) report differentiation of mouse embryonic stem cells to insulin-secreting structures similar to pancreatic islets. Soria et al. (Diabetes 49:157, 2000) report that insulin-secreting cells derived from mouse embryonic stem cells normalize glycemia in streptozotocin-induced diabetic mice.

In one example, Hori et al. (PNAS 99: 16105, 2002) disclose that treatment of mouse embryonic stem cells with inhibitors of phosphoinositide 3-kinase (LY294002) produced cells that resembled β cells.

In another example, Blyszczuk et al. (PNAS 100:998, 2003) reports the generation of insulin-producing cells from mouse embryonic stem cells constitutively expressing Pax4.

Micallef *et al.* reports that retinoic acid can regulate the commitment of embryonic stem cells to form PDX1 positive pancreatic endoderm. Retinoic acid is most effective at inducing PDX1 expression when added to cultures at day four of embryonic stem cell differentiation during a period corresponding to the end of gastrulation in the embryo (Diabetes 54:301, 2005).

Miyazaki *et al.* reports a mouse embryonic stem cell line over-expressing Pdx1. Their results show that exogenous Pdx1 expression clearly enhanced the expression of insulin, somatostatin, glucokinase, neurogenin3, p48, Pax6, and HNF6 genes in the resulting differentiated cells (Diabetes 53: 1030, 2004).

Skoudy *et al.* reports that activin A (a member of the TGF-β superfamily) upregulates the expression of exocrine pancreatic genes (p48 and amylase) and endocrine genes (Pdx1, insulin, and glucagon) in mouse embryonic stem cells. The maximal effect was observed using InM activin A. They also observed that the expression level of insulin and Pdx1 mRNA was not affected by retinoic acid; however, 3nM FGF7 treatment resulted in an increased level of the transcript for Pdx1 (Biochem. J. 379: 749, 2004).

Shiraki *et al.* studied the effects of growth factors that specifically enhance differentiation of embryonic stem cells into PDX1 positive cells. They observed that TGF-β2 reproducibly yielded a higher proportion of PDX1 positive cells (Genes Cells. 2005 Jun; 10(6): 503-16.).

Gordon *et al.* demonstrated the induction of brachyury [positive]/HNF3 beta [positive] endoderm cells from mouse embryonic stem cells in the absence of serum and in the presence of activin along with an inhibitor of Wnt signaling (US 2006/0003446A1).

Gordon et al. (PNAS, Vol 103, page 16806, 2006) states "Wnt and TGF-beta/ nodal/ activin signaling simultaneously were required for the generation of the anterior primitive streak".

However, the mouse model of embryonic stem cell development may not exactly mimic the developmental program in higher mammals, such as, for example, humans.

Thomson *et al.* isolated embryonic stem cells from human blastocysts (Science 282:114, 1998). Concurrently, Gearhart and coworkers derived human embryonic germ (hEG) cell lines from fetal gonadal tissue (Shamblott et al., Proc. Natl. Acad. Sci. USA 95:13726, 1998). Unlike mouse embryonic stem cells, which can be prevented from differentiating simply by culturing with Leukemia Inhibitory Factor (LIF), human embryonic stem cells must be maintained under very special conditions (U.S. Pat. No. 6,200,806; WO 99/20741; WO 01/51616).

D'Amour *et al.* describes the production of enriched cultures of human embryonic stem cell-derived definitive endoderm in the presence of a high concentration of activin and low serum (Nature Biotechnology 2005). Transplanting these cells under the kidney capsule of mice resulted in differentiation into more mature cells with characteristics of some endodermal organs. Human embryonic stem cell-derived definitive endoderm cells can be further differentiated into PDX1 positive cells after addition of FGF-10 (US 2005/0266554A1).

D'Amour et al. (Nature Biotechnology - 24, 1392 - 1401 (2006)) states: "We have developed a differentiation process that converts human embryonic stem (hES) cells to endocrine cells capable of synthesizing the pancreatic hormones insulin, glucagon, somatostatin, pancreatic polypeptide and ghrelin. This process mimics *in vivo* pancreatic organogenesis by directing cells through stages resembling definitive endoderm, gut-tube endoderm, pancreatic endoderm and endocrine precursor en route to cells that express endocrine hormones".

In another example, Fisk *et al.* reports a system for producing pancreatic islet cells from human embryonic stem cells (US2006/0040387A1). In this case, the differentiation pathway was divided into three stages. Human embryonic stem cells were first differentiated to endoderm using a combination of sodium butyrate and activin A. The cells were then cultured with TGF-β antagonists such as Noggin in combination with EGF or betacellulin to generate PDX1 positive cells. The terminal differentiation was induced by nicotinamide.

In one example, Benvenistry *et al.* states: "We conclude that over-expression of PDX1 enhanced expression of pancreatic enriched genes, induction of insulin expression may require additional signals that are only present *in vivo"* (Benvenistry et al, Stem Cells 2006; 24:1923-1930).

In another example, US2008/0241107A1 claims a method for producing a cell that secretes insulin comprising: a) obtaining a cell that does not produce insulin; and, b) incubating the cell with media containing high glucose, wherein the cell secretes insulin.

WO2009/018453 discloses a method for the formation of pancreatic endoderm, pancreatic hormone expressing cells and pancreatic hormone secreting cells.

WO01/23528 discloses use of islet-producing stem cells, islet progenitor cells (IPCs) and IPC-derived islets (IdIs) for implantation into a mammal for in vivo therapy of diabetes.

WO2010/057039 discloses methods for encapsulating pancreatic progenitors in a biocompatible semi-permeable encapsulating device.

Foster *et al.* disclose the differentiation of transplanted microencapsulated fetal pancreatic cells (Transplantation. 2007 Jun 15;83(11):1440-8).

Therefore, there still remains a significant need to develop conditions for establishing pluripotent stem cell lines that can be expanded to address the current clinical needs, while retaining the potential to differentiate into pancreatic endocrine cells, pancreatic hormone expressing cells, or pancreatic hormone secreting cells. An alternative approach to improve the efficiency of differentiating human embryonic stem cells toward pancreatic endocrine cells has been taken.

### SUMMARY

In one embodiment, the present invention provides a population of encapsulated pancreatic endocrine precursor cells for use in a method for lowering blood glucose levels in an animal by transplanting the population of encapsulated pancreatic endocrine precursor cells into an animal, as defined in the claims.

### BRIEF DESCRIPTION OF THE DRAWINGS

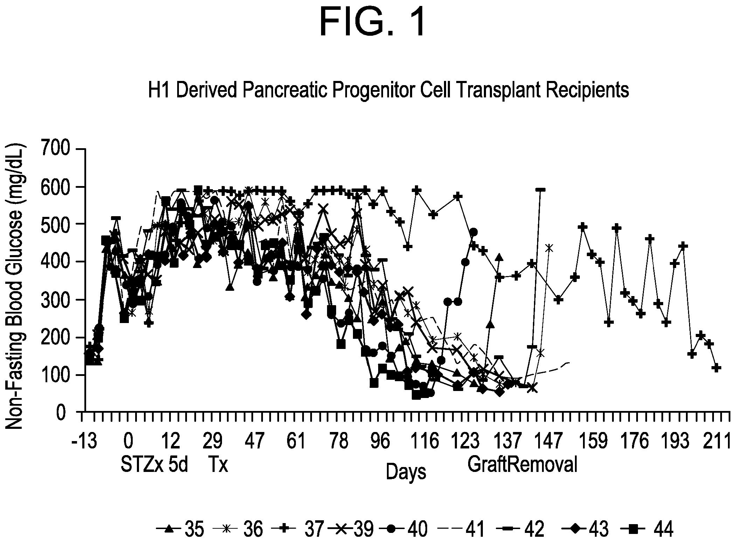

Figure 1 shows blood glucose levels in SCID mice that were rendered diabetic by 5 injections of streptozotocin and then transplanted under the kidney capsule with differentiated human ES cells (stage 4) on day 0. Blood glucose tracking of the following several months revealed a gradual decline in hyperglycemia to pre-diabetic levels. Subsequent kidney removal resulted in a rapid recurrence of diabetes.

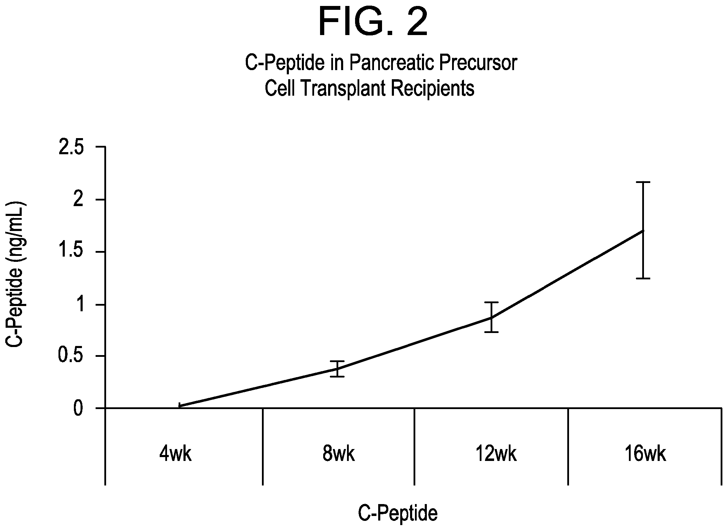

Figure 2 shows Human C-peptide measurements in plasma samples at the indicated weeks post transplant show progressive increases commensurate with the fall in blood glucose levels.

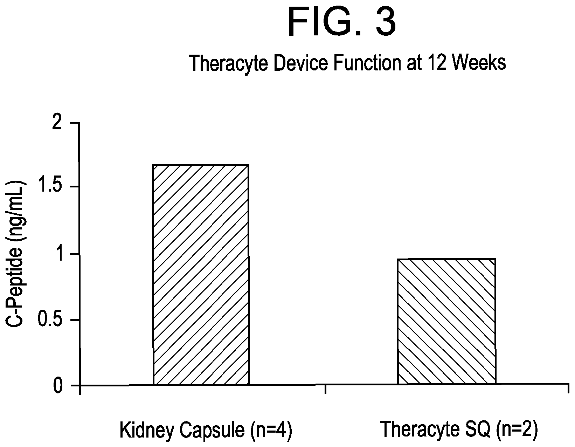

Figure 3 shows comparable C-peptide levels obtained in recipients of cells transplanted under the kidney capsule or subcutaneously within TheraCyte devices.

### DETAILED DESCRIPTION

For clarity of disclosure, and not by way of limitation, the detailed description of the invention is divided into the following subsections that describe or illustrate certain features, embodiments or applications of the present invention.

### Definitions

Stem cells are undifferentiated cells defined by their ability at the single cell level to both self-renew and differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages but all within a particular tissue, organ, or physiological system (for example, hematopoietic stem cells (HSC) can produce progeny that include HSC (self- renewal), blood cell restricted oligopotent progenitors, and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g. spermatogenic stem cells).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

"Cells expressing markers characteristic of the definitive endoderm lineage", or "Stage 1 cells", or "Stage 1", as used herein, refers to cells expressing at least one of the following markers: SOX-17, GATA4, HNF3 beta, GSC, CER1, Nodal, FGF8, Brachyury, Mix-like homeobox protein, FGF4 CD48, eomesodermin (EOMES), DKK4, FGF17, GATA6, CXCR4, C-Kit, CD99, or OTX2. Cells expressing markers characteristic of the definitive endoderm lineage include primitive streak precursor cells, primitive streak cells, mesendoderm cells and definitive endoderm cells.

"Cells expressing markers characteristic of the pancreatic endoderm lineage", as used herein, refers to cells expressing at least one of the following markers: PDX1, HNF-1 beta, PTF1 alpha, HNF6, or HB9. Cells expressing markers characteristic of the pancreatic endoderm lineage include pancreatic endoderm cells, primitive gut tube cells, and posterior foregut cells.

"Cells expressing markers characteristic of the pancreatic endocrine lineage", as used herein, refers to cells expressing at least one of the following markers: NEUROD, ISL1, PDX1, NKX6.1, MAFB, insulin, glucagon, or somatostatin. Cells expressing markers characteristic of the pancreatic endocrine lineage include pancreatic endocrine cells, pancreatic hormone expressing cells, and pancreatic hormone secreting cells, and cells of the β-cell lineage.

"Definitive endoderm", as used herein, refers to cells which bear the characteristics of cells arising from the epiblast during gastrulation and which form the gastrointestinal tract and its derivatives. Definitive endoderm cells express the following markers: HNF3 beta, GATA4, SOX17, Cerberus, OTX2, goosecoid, C-Kit, CD99, and MIXL1.

"Markers", as used herein, are nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

"Pancreatic endocrine cell", or "pancreatic hormone expressing cell", as used herein, refers to a cell capable of expressing at least one of the following hormones: insulin, glucagon, somatostatin, and pancreatic polypeptide.

"Pancreatic endocrine precursor cell", as used herein refers to a multipotent cell of the definitive endoderm lineage that expresses NGN3 and which can further differentiate into cells of the endocrine system including, but not limited to, pancreatic islet hormone-expressing cells. Endocrine precursor cells cannot differentiate into as many different cell, tissue and/or organ types as compared to less specifically differentiated definitive endoderm lineage cells, such as PDX1 positive pancreatic endoderm cells.

"Pancreatic hormone producing cell", as used herein, refers to a cell capable of producing at least one of the following hormones: insulin, glucagon, somatostatin, and pancreatic polypeptide.

"Pancreatic hormone secreting cell" as used herein, refers to a cell capable of secreting at least one of the following hormones: insulin, glucagon, somatostatin, and pancreatic polypeptide.

### Isolation, Expansion and Culture of Pluripotent Stem Cells

### Characterization of Pluripotent Stem Cells

Pluripotent stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of pluripotent stem cells *in vitro* results in the loss of SSEA-4, Tra- 1-60, and Tra-1-81 expression (if present) and increased expression of SSEA-1. Undifferentiated pluripotent stem cells typically have alkaline phosphatase activity, which can be detected by fixing the cells with 4% paraformaldehyde, and then developing with Vector Red as a substrate, as described by the manufacturer (Vector Laboratories, Burlingame Calif.) Undifferentiated pluripotent stem cells also typically express Oct-4 and TERT, as detected by RT-PCR.

Another desirable phenotype of propagated pluripotent stem cells is a potential to differentiate into cells of all three germinal layers: endoderm, mesoderm, and ectoderm tissues. Pluripotency of pluripotent stem cells can be confirmed, for example, by injecting cells into severe combined immunodeficient (SCID) mice, fixing the teratomas that form using 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated pluripotent stem cell lines may be karyotyped using a standard G-banding technique and compared to published karyotypes of the corresponding primate species. It is desirable to obtain cells that have a "normal karyotype," which means that the cells are euploid, wherein all human chromosomes are present and not noticeably altered.

### Sources of Pluripotent Stem Cells

The invention does not use cells which have been obtained through destruction of a human embryo. The types of pluripotent stem cells that may be used include established lines of pluripotent cells derived from tissue formed after gestation, including non-human pre-embryonic tissue (such as, for example, a blastocyst), non-human embryonic tissue, or fetal tissue taken any time during gestation, typically but not necessarily before approximately 10-12 weeks gestation. Also contemplated is use of the compositions of this disclosure during the initial establishment or stabilization of such cells, in which case the source cells would be primary pluripotent cells taken directly from the source tissues. Also suitable are cells taken from a pluripotent stem cell population already cultured in the absence of feeder cells. Also contemplated is use of the compositions of this disclosure during the initial establishment or stabilization of such cells, in which case the source cells would be primary pluripotent cells taken directly from the source tissues. Also suitable are cells taken from a pluripotent stem cell population already cultured in the absence of feeder cells. Also suitable are mutant human embryonic stem cell lines, such as, for example, reference cell line BG01v (BresaGen, Athens, GA).

### Culture of Pluripotent Stem Cells

In one embodiment, pluripotent stem cells are typically cultured on a layer of feeder cells that support the pluripotent stem cells in various ways. Alternatively, pluripotent stem cells are cultured in a culture system that is essentially free of feeder cells, but nonetheless supports proliferation of pluripotent stem cells without undergoing substantial differentiation. The growth of pluripotent stem cells in feeder-free culture without differentiation is supported using a medium conditioned by culturing previously with another cell type. Alternatively, the growth of pluripotent stem cells in feeder-free culture without differentiation is supported using a chemically defined medium.

For reference, Reubinoff et al (Nature Biotechnology 18: 399 - 404 (2000)) and Thompson et al (Science 6 November 1998: Vol. 282. no. 5391, pp. 1145 - 1147) disclose the culture of pluripotent stem cell lines from human blastocysts using a mouse embryonic fibroblast feeder cell layer.

Also for reference, Richards et al, (Stem Cells 21: 546-556, 2003) evaluated a panel of eleven different human adult, fetal and neonatal feeder cell layers for their ability to support human pluripotent stem cell culture. Richards *et al,* states: "human embryonic stem cell lines cultured on adult skin fibroblast feeders retain human embryonic stem cell morphology and remain pluripotent".

For reference, US20020072117 discloses cell lines that produce media that support the growth of primate pluripotent stem cells in feeder-free culture. The cell lines employed are mesenchymal and fibroblast-like cell lines obtained from embryonic tissue or differentiated from embryonic stem cells. US20020072117 also discloses the use of the cell lines as a primary feeder cell layer.

In another reference example, Wang et al (Stem Cells 23: 1221-1227, 2005) discloses methods for the long-term growth of human pluripotent stem cells on feeder cell layers derived from human embryonic stem cells.

In another reference example, Stojkovic et al (Stem Cells 2005 23: 306-314, 2005) disclose a feeder cell system derived from the spontaneous differentiation of human embryonic stem cells.

In a further reference example, Miyamoto et al (Stem Cells 22: 433-440, 2004) disclose a source of feeder cells obtained from human placenta.

Amit et al (Biol. Reprod 68: 2150-2156, 2003) discloses a feeder cell layer derived from human foreskin.

In another example, Inzunza et al (Stem Cells 23: 544-549, 2005) disclose a feeder cell layer from human postnatal foreskin fibroblasts.

US6642048 discloses media that support the growth of primate pluripotent stem (pPS) cells in feeder-free culture, and cell lines useful for production of such media. US6642048 states: "This invention includes mesenchymal and fibroblast-like cell lines obtained from embryonic tissue or differentiated from embryonic stem cells. Methods for deriving such cell lines, processing media, and growing stem cells using the conditioned media are described and illustrated in this disclosure."

In another example, WO2005014799 discloses conditioned medium for the maintenance, proliferation and differentiation of mammalian cells. WO2005014799 states: "The culture medium produced in accordance with the present invention is conditioned by the cell secretion activity of murine cells; in particular, those differentiated and immortalized transgenic hepatocytes, named MMH (Met Murine Hepatocyte)."

In another example, Xu et al (Stem Cells 22: 972-980, 2004) discloses conditioned medium obtained from human embryonic stem cell derivatives that have been genetically modified to over express human telomerase reverse transcriptase.

In another example, US20070010011 discloses a chemically defined culture medium for the maintenance of pluripotent stem cells.

An alternative culture system employs serum-free medium supplemented with growth factors capable of promoting the proliferation of embryonic stem cells. For example, Cheon et al (BioReprod DOI:10.1095/biolreprod.105.046870, October 19, 2005) disclose a feeder-free, serum-free culture system in which embryonic stem cells are maintained in unconditioned serum replacement (SR) medium supplemented with different growth factors capable of triggering embryonic stem cell self-renewal.

In another example, Levenstein et al (Stem Cells 24: 568-574, 2006) disclose methods for the long-term culture of human embryonic stem cells in the absence of fibroblasts or conditioned medium, using media supplemented with bFGF.

In another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

In another example, US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells.

In another example, US6800480 states "In one embodiment, a cell culture medium for growing primate-derived primordial stem cells in a substantially undifferentiated state is provided which includes a low osmotic pressure, low endotoxin basic medium that is effective to support the growth of primate-derived primordial stem cells. The basic medium is combined with a nutrient serum effective to support the growth of primate-derived primordial stem cells and a substrate selected from the group consisting of feeder cells and an extracellular matrix component derived from feeder cells. The medium further includes non-essential amino acids, an anti-oxidant, and a first growth factor selected from the group consisting of nucleosides and a pyruvate salt."

In another example, US20050244962 states: "In one aspect the invention provides a method of culturing primate embryonic stem cells. One cultures the stem cells in a culture essentially free of mammalian fetal serum (preferably also essentially free of any animal serum) and in the presence of fibroblast growth factor that is supplied from a source other than just a fibroblast feeder layer. In a preferred form, the fibroblast feeder layer, previously required to sustain a stem cell culture, is rendered unnecessary by the addition of sufficient fibroblast growth factor."

In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In another example, WO2005086845 discloses a method for maintenance of an undifferentiated stem cell, said method comprising exposing a stem cell to a member of the transforming growth factor-beta (TGF-β) family of proteins, a member of the fibroblast growth factor (FGF) family of proteins, or nicotinamide (NIC) in an amount sufficient to maintain the cell in an undifferentiated state for a sufficient amount of time to achieve a desired result.

The pluripotent stem cells may be plated onto a suitable culture substrate. In one embodiment, the suitable culture substrate is an extracellular matrix component, such as, for example, those derived from basement membrane or that may form part of adhesion molecule receptor-ligand couplings. In one embodiment, the suitable culture substrate is MATRIGFL® (Becton Dickenson). MATRIGFL® is a soluble preparation from Engelbreth-Holm Swarm tumor cells that gels at room temperature to form a reconstituted basement membrane.

Other extracellular matrix components and component mixtures are suitable as an alternative. Depending on the cell type being proliferated, this may include laminin, fibronectin, proteoglycan, entactin, heparan sulfate, and the like, alone or in various combinations.

The pluripotent stem cells may be plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

Suitable culture media may be made from the following components, such as, for example, Dulbecco's modified Eagle's medium (DMEM), Gibco # 11965-092; Knockout Dulbecco's modified Eagle's medium (KO DMEM), Gibco #10829-018; Ham's F12/50% DMEM basal medium; 200 mM L-glutamine, Gibco # 15039-027; non-essential amino acid solution, Gibco 11140-050; β-mercaptoethanol, Sigma # M7522; human recombinant basic fibroblast growth factor (bFGF), Gibco # 13256-029.

### Formation of Pancreatic Endocrine Precursor Cells

In one embodiment, the present disclosure provides a method for producing pancreatic endocrine precursor cells, comprising the steps of:
a. Culturing pluripotent stem cells,
b. Differentiating the pluripotent stem cells into cells expressing markers characteristic of the definitive endoderm lineage,
c. Differentiating the cells expressing markers characteristic of the definitive endoderm lineage into cells expressing markers characteristic of the pancreatic endoderm lineage, and
d. Differentiating the expressing markers characteristic of the pancreatic endoderm lineage into pancreatic endocrine precursor cells.

Suitable for use in the present invention are cells that express at least one of the following markers characteristic of pluripotent cells: ABCG2, CRIPTO, CD9, FOXD3, Connexin43, Connexin45, OCT4, SOX2, Nanog, hTERT, UTF1, ZFP42, SSEA-3, SSEA-4, Tra 1-60, Tra 1-81.

Markers characteristic of the definitive endoderm lineage are selected from the group consisting of SOX17, GATA4, HNF3 beta, GSC, CER1, Nodal, FGF8, Brachyury, Mix-like homeobox protein, FGF4 CD48, eomesodermin (EOMES), DKK4, FGF17, GATA6, CXCR4, C-Kit, CD99, and OTX2. Suitable for use in the present invention is a cell that expresses at least one of the markers characteristic of the definitive endoderm lineage. In one aspect of the present invention, a cell expressing markers characteristic of the definitive endoderm lineage is a primitive streak precursor cell. In an alternate aspect, a cell expressing markers characteristic of the definitive endoderm lineage is a mesendoderm cell. In an alternate aspect, a cell expressing markers characteristic of the definitive endoderm lineage is a definitive endoderm cell.

Markers characteristic of the pancreatic endoderm lineage are selected from the group consisting of PDX1, HNF1 beta, HNF6, HB9 and PROX1. Suitable for use in the present invention is a cell that expresses at least one of the markers characteristic of the pancreatic endoderm lineage. In one aspect of the present invention, a cell expressing markers characteristic of the pancreatic endoderm lineage is a pancreatic endoderm cell.

Markers characteristic of pancreatic endocrine precursor cells are selected from the group consisting of NGN3, NKX6.1, NeuroD, ISL1, PDX1, PAX4, NKX2.2, or ARX. Suitable for use in the present invention is a cell that expresses at least one of the markers characteristic of pancreatic endocrine precursor cells, as defined in the claims.

### Formation of Cells Expressing Markers Characteristic of the Definitive Endoderm Lineage

Pluripotent stem cells may be differentiated into cells expressing markers characteristic of the definitive endoderm lineage by any method in the art or by any method proposed in this invention.

For example, pluripotent stem cells may be differentiated into cells expressing markers characteristic of the definitive endoderm lineage according to the methods disclosed in D'Amour et al, Nature Biotechnology 23, 1534 - 1541 (2005).

For example, pluripotent stem cells may be differentiated into cells expressing markers characteristic of the definitive endoderm lineage according to the methods disclosed in Shinozaki et al, Development 131, 1651 - 1662 (2004).

For example, pluripotent stem cells may be differentiated into cells expressing markers characteristic of the definitive endoderm lineage according to the methods disclosed in McLean et al, Stem Cells 25, 29 - 38 (2007).

For example, pluripotent stem cells may be differentiated into cells expressing markers characteristic of the definitive endoderm lineage according to the methods disclosed in D'Amour et al, Nature Biotechnology 24, 1392 - 1401 (2006).

For example, pluripotent stem cells may be differentiated into cells expressing markers characteristic of the definitive endoderm lineage by culturing the pluripotent stem cells in medium containing activin A in the absence of serum, then culturing the cells with activin A and serum, and then culturing the cells with activin A and serum of a different concentration. An example of this method is disclosed in Nature Biotechnology 23, 1534 - 1541 (2005).

For example, pluripotent stem cells may be differentiated into cells expressing markers characteristic of the definitive endoderm lineage by culturing the pluripotent stem cells in medium containing activin A in the absence of serum, then culturing the cells with activin A with serum of another concentration. An example of this method is disclosed in D' Amour et al, Nature Biotechnology, 2005.

For example, pluripotent stem cells may be differentiated into cells expressing markers characteristic of the definitive endoderm lineage by culturing the pluripotent stem cells in medium containing activin A and a Wnt ligand in the absence of serum, then removing the Wnt ligand and culturing the cells with activin A with serum. An example of this method is disclosed in Nature Biotechnology 24, 1392 - 1401 (2006).

For example, pluripotent stem cells may be differentiated into cells expressing markers characteristic of the definitive endoderm lineage by treating the pluripotent stem cells according to the methods disclosed in US patent application Ser. No. 11/736,908, assigned to LifeScan, Inc.

For example, pluripotent stem cells may be differentiated into cells expressing markers characteristic of the definitive endoderm lineage by treating the pluripotent stem cells according to the methods disclosed in US patent application Ser. No. 11/779,311, assigned to LifeScan, Inc.

For example, pluripotent stem cells may be differentiated into cells expressing markers characteristic of the definitive endoderm lineage by treating the pluripotent stem cells according to the methods disclosed in US patent application Ser. No. 60/990,529.

For example, pluripotent stem cells may be differentiated into cells expressing markers characteristic of the definitive endoderm lineage by treating the pluripotent stem cells according to the methods disclosed in US patent application Ser. No. 61/076,889.

For example, pluripotent stem cells may be differentiated into cells expressing markers characteristic of the definitive endoderm lineage by treating the pluripotent stem cells according to the methods disclosed in US patent application Ser. No. 61/076,900.

For example, pluripotent stem cells may be differentiated into cells expressing markers characteristic of the definitive endoderm lineage by treating the pluripotent stem cells according to the methods disclosed in US patent application Ser. No. 61/076,908.

For example, pluripotent stem cells may be differentiated into cells expressing markers characteristic of the definitive endoderm lineage by treating the pluripotent stem cells according to the methods disclosed in US patent application Ser. No. 61/076,915.

### Characterization of Cells Expressing Markers Characteristic of the Definitive Endoderm Lineage

Formation of cells expressing markers characteristic of the definitive endoderm lineage may be determined by testing for the presence of the markers before and after following a particular protocol. Pluripotent stem cells typically do not express such markers. Thus, differentiation of pluripotent cells is detected when cells begin to express them.

The efficiency of differentiation may be determined by exposing a treated cell population to an agent (such as an antibody) that specifically recognizes a protein marker expressed by cells expressing markers characteristic of the definitive endoderm lineage.

Methods for assessing expression of protein and nucleic acid markers in cultured or isolated cells are standard in the art. These include quantitative reverse transcriptase polymerase chain reaction (RT-PCR), Northern blots, *in situ* hybridization (see, e.g., Current Protocols in Molecular Biology (Ausubel et al., eds. 2001 supplement)), and immunoassays such as immunohistochemical analysis of sectioned material, Western blotting, and for markers that are accessible in intact cel*ls*, *flow* cytometry analysis (FACS) (see, e.g., Harlow and Lane, Using Antibodies: A Laboratory Manual, New York: Cold Spring Harbor Laboratory Press (1998)).

Characteristics of pluripotent stem cells are well known to those skilled in the art, and additional characteristics of pluripotent stem cells continue to be identified. Pluripotent stem cell markers include, for example, the expression of one or more of the following: ABCG2, CRIPTO, FOXD3, Connexin43, *Connexin*45, OCT4, SOX2, Nanog, hTERT, UTF1, ZFP42, SSEA-3, SSEA-4, Tra 1-60, Tra 1-81.

After treating pluripotent stem cells with the methods of the present disclosure, the *differentiated* cells may be purified by exposing a treated cell population to an agent (*such a*s an antibody) that specifically recognizes a p*rotein* marker, such as CXCR4, expressed by cells expressing markers characteristic *of* the definitive endoderm lineage.

### Formation of Cells Expressing Markers Characteristic of the Pancreatic Endoderm Lineage from Cells Expressing Markers Characteristic of the Definitive Endoderm Lineage

Cells expressing markers characteristic of the defin*itive* endoderm lineage may be differentiated into cells expressing markers characteristic of the pancreatic endoderm lineage by any method in the art or by any method proposed in this invention, as defined in the claims.

For example, cells expressing markers characteristic of the definitive endoderm lineage may be differentiated into cells expressing markers characteristic of the pancreatic endoderm lineage according to the methods disclosed in D'Amour et al, Nature Biotechnology 24, 1392 - 1401 (2006).

For example, cells expressing markers characteristic of the definitive endoderm lineage are further differentiated into cells expressing markers characteristic of the pancreatic endoderm lineage, by treating the cells expressing markers characteristic of the definitive endoderm lineage with a fibroblast growth factor and the hedgehog signaling pathway inhibitor KAAD-cyclopamine, then removing the medium containing the fibroblast growth factor and KAAD-cyclopamine and subsequently culturing the cells in medium containing retinoic acid, a fibroblast growth factor and KAAD-cyclopamine. An example of this method is disclosed in Nature Biotechnology 24, 1392 - 1401 (2006).

In one aspect of the present invention, cells expressing markers characteristic of the definitive endoderm lineage are further differentiated into cells expressing markers characteristic of the pancreatic endoderm lineage, by treating the cells expressing markers characteristic of the definitive endoderm lineage with retinoic acid and at least one fibroblast growth factor for a period of time, according to the methods disclosed in US patent application Ser. No. 11/736,908, assigned to LifeScan, Inc.

In one aspect of the present disclosure, cells expressing markers characteristic of the definitive endoderm lineage are further differentiated into cells expressing markers characteristic of the pancreatic endoderm lineage, by treating the cells expressing markers characteristic of the definitive endoderm lineage with retinoic acid and at least one fibroblast growth factor for a period of time, according to the methods disclosed in US patent application Ser. No. 11/779,311, assigned to LifeScan, Inc.

In one aspect of the present invention, cells expressing markers characteristic of the definitive endoderm lineage are further differentiated into cells expressing markers characteristic of the pancreatic endoderm lineage, by treating the cells expressing markers characteristic of the definitive endoderm lineage according to the methods disclosed in US patent application Ser. No. 60/990,529.

### Characterization of Cells Expressing Markers Characteristic of the Pancreatic Endoderm Lineage

Markers characteristic of the pancreatic endoderm lineage are well known to those skilled in the art, and additional markers characteristic of the pancreatic endoderm lineage continue to be identified. These markers can be used to confirm that the cells treated in accordance with the present invention have differentiated to acquire the properties characteristic of the pancreatic endoderm lineage. Pancreatic endoderm lineage specific markers include the expression of one or more transcription factors such as, for example, HLXB9, PTF1 alpha, PDX1, HNF6, HNF-1 beta.

The efficiency of differentiation may be determined by exposing a treated cell population to an agent (such as an antibody) that specifically recognizes a protein marker expressed by cells expressing markers characteristic of the pancreatic endoderm lineage.

Methods for assessing expression of protein and nucleic acid markers in cultured or isolated cells are standard in the art. These include quantitative reverse transcriptase polymerase chain reaction (RT-PCR), Northern blots, *in situ* hybridization (see, e.g., Current Protocols in Molecular Biology (Ausubel et al., eds. 2001 supplement)), and immunoassays such as immunohistochemical analysis of sectioned material, Western blotting, and for markers that are accessible in intact cells, flow cytometry analysis (FACS) (see, e.g., Harlow and Lane, Using Antibodies: A Laboratory Manual, New York: Cold Spring Harbor Laboratory Press (1998)).

### Formation of Pancreatic Endocrine Precursor Cells from Cells Expressing Markers Characteristic of the Pancreatic Endoderm Lineage

In one aspect of the present invention, cells expressing markers characteristic of the pancreatic endoderm lineage are differentiated into pancreatic endocrine precursor cells, by culturing the cells expressing markers characteristic of the pancreatic endoderm lineage in medium supplemented with a factor capable of inhibiting BMP and a TGF-β receptor I kinase inhibitor, as defined in the claims.

In one embodiment, the factor capable of inhibiting BMP is noggin. Noggin may be used at a concentration from about 100pg/ml to about 500µg/ml. In one embodiment, noggin is used at a concentration of 100ng/ml.

In one embodiment, the TGF-β receptor I kinase inhibitor is ALK5 inhibitor II (Calbiochem, Ca). ALK5 inhibitor II may be used at a concentration from about 0.1µM to about 10µM. In one embodiment, ALK5 inhibitor II is used at a concentration of 1µM.

In one embodiment, the medium is DMEM containing 4500mg/l glucose and 1% B27.

In one embodiment, the cells are cultured in the culture medium for about four days.

The efficiency of differentiation may be determined by exposing a treated cell population to an agent (such as an antibody) that specifically recognizes a protein marker expressed by pancreatic endocrine precursor cells.

Methods for assessing expression of protein and nucleic acid markers in cultured or isolated cells are standard in the art. These include quantitative reverse transcriptase polymerase chain reaction (RT-PCR), Northern blots, *in situ* hybridization (see, e.g., Current Protocols in Molecular Biology (Ausubel et al., eds. 2001 supplement)), and immunoassays such as immunohistochemical analysis of sectioned material, Western blotting, and for markers that are accessible in intact cells, flow cytometry analysis (FACS) (see, e.g., Harlow and Lane, Using Antibodies: A Laboratory Manual, New York: Cold Spring Harbor Laboratory Press (1998)).

Characteristics of pluripotent stem cells are well known to those skilled in the art, and additional characteristics of pluripotent stem cells continue to be identified. Pluripotent stem cell markers include, for example, the expression of one or more of the following: ABCG2, CRIPTO, FOXD3, Connexin43, Connexin45, OCT4, SOX2, Nanog, hTERT, UTF1, ZFP42, SSEA-3, SSEA-4, Tra 1-60, Tra 1-81.

After treating pluripotent stem cells with the methods of the present disclosure, the differentiated cells may be purified by exposing a treated cell population to an agent (such as an antibody) that specifically recognizes a protein marker, such as CXCR4, expressed by cells expressing markers characteristic of the pancreatic endoderm lineage.

Markers characteristic of the pancreatic endoderm lineage are selected from the group consisting of PDX1, HNF-1 beta, PTF1 alpha, HNF6, HB9 and PROX1. Suitable for use in the present invention is a cell that expresses at least one of the markers characteristic of the pancreatic endoderm lineage. In one aspect of the present invention, a cell expressing markers characteristic of the pancreatic endoderm lineage is a pancreatic endoderm cell.

Markers characteristic of pancreatic endocrine precursor cells are selected from the group consisting of NGN3, NKX6.1, NEUROD, ISL1, PDX1, PAX4, NKX2.2, PAX6 or ARX.

### Formation of Cells Expressing Markers Characteristic of the Pancreatic Endocrine Lineage from Pancreatic Endocrine Precursor Cells

In one embodiment, pancreatic endocrine precursor cells, as defined in the claims, may be further differentiated into cells expressing markers characteristic of the pancreatic endocrine lineage.

Pancreatic endocrine precursor cells may be differentiated into cells expressing markers characteristic of the pancreatic endocrine lineage by any method in the art or by any method proposed in this invention.

For example, pancreatic endocrine precursor cells, as defined in the claims, are further differentiated into cells expressing markers characteristic of the pancreatic endocrine lineage, by culturing the pancreatic endocrine precursor cells in medium containing exendin 4, then removing the medium containing exendin 4 and subsequently culturing the cells in medium containing exendin 1, IGF1 and HGF. An example of this method is disclosed in D' Amour et al, Nature Biotechnology, 2006.

For example, pancreatic endocrine precursor cells obtained according to the methods of the present disclosure are further differentiated into cells expressing markers characteristic of the pancreatic endocrine lineage, by culturing the pancreatic endocrine precursor cells in medium containing DAPT (Sigma-Aldrich, MO) and exendin 4. An example of this method is disclosed in D' Amour et al, Nature Biotechnology, 2006.

For example, pancreatic endocrine precursor cells obtained according to the methods of the present disclosure are further differentiated into cells expressing markers characteristic of the pancreatic endocrine lineage, by culturing the pancreatic endocrine precursor cells in medium containing exendin 4. An example of this method is disclosed in D' Amour et al, Nature Biotechnology, 2006.

For example, cells pancreatic endocrine precursor cells obtained according to the methods of the present disclosure are further differentiated into cells expressing markers characteristic of the pancreatic endocrine lineage, by treating the pancreatic endocrine precursor cells with a factor that inhibits the Notch signaling pathway, according to the methods disclosed in US patent application Ser. No. 11/736,908, assigned to LifeScan, Inc.

For example, pancreatic endocrine precursor cells obtained according to the methods of the present disclosure are further differentiated into cells expressing markers characteristic of the pancreatic endocrine lineage, by treating the pancreatic endocrine precursor cells with a factor that inhibits the Notch signaling pathway, according to the methods disclosed in US patent application Ser. No. 11/779,311, assigned to LifeScan, Inc.

For example, pancreatic endocrine precursor cells obtained according to the methods of the present disclosure are further differentiated into cells expressing markers characteristic of the pancreatic endocrine lineage, by treating the pancreatic endocrine precursor cells with a factor that inhibits the Notch signaling pathway, according to the methods disclosed in US patent application Ser. No. 60/953,178, assigned to LifeScan, Inc.

For example, pancreatic endocrine precursor cells obtained according to the methods of the present disclosure are further differentiated into cells expressing markers characteristic of the pancreatic endocrine lineage, by treating the pancreatic endocrine precursor cells with a factor that inhibits the Notch signaling pathway, according to the methods disclosed in US patent application Ser. No. 60/990,529, assigned to LifeScan, Inc.

Markers characteristic of the pancreatic endocrine lineage are selected from the group consisting of NEUROD, ISL1, PDX1, NKX6.1, PAX4, PAX6, NGN3, and NKX2.2. In one embodiment, a pancreatic endocrine cell is capable of expressing at least one of the following hormones: insulin, glucagon, somatostatin, and pancreatic polypeptide. Suitable for use in the present invention is a cell that expresses at least one of the markers characteristic of the pancreatic endocrine lineage. In one aspect of the present invention, a cell expressing markers characteristic of the pancreatic endocrine lineage is a pancreatic endocrine cell. The pancreatic endocrine cell may be a pancreatic hormone-expressing cell. Alternatively, the pancreatic endocrine cell may be a pancreatic hormone-secreting cell.

In one aspect of the present disclosure, the pancreatic endocrine cell is a cell expressing markers characteristic of the β cell lineage. A cell expressing markers characteristic of the β cell lineage expresses PDX1 and at least one of the following transcription factors: NGN-3, NKX2.2, NKX6.1, NEUROD, ISL1, HNF3 beta, MAFA, PAX4, and PAX6. In one aspect of the present invention, a cell expressing markers characteristic of the β cell lineage is a β cell.

### Therapies

In one aspect, the present disclosure provides a method for treating a patient suffering from, or at risk of developing, Type1 diabetes. In one embodiment, the method involves culturing pluripotent stem cells, differentiating the pluripotent stem cells *in vitro* into a β-cell lineage, and implanting the cells of a β-cell lineage into a patient. In an alternate embodiment, the method involves culturing pluripotent stem cells, differentiating the pluripotent stem cells *in vitro* into pancreatic endocrine precursor cells, and implanting the pancreatic endocrine precursor cells into a patient.

In yet another aspect, this disclosure provides a method for treating a patient suffering from, or at risk of developing, Type 2 diabetes. In one embodiment, the method involves culturing pluripotent stem cells, differentiating the pluripotent stem cells *in vitro* into a β-cell lineage, and implanting the cells of a β-cell lineage into a patient. In an alternate embodiment, the method involves culturing pluripotent stem cells, differentiating the pluripotent stem cells *in vitro* into pancreatic endocrine precursor cells, and implanting the pancreatic endocrine precursor cells into a patient.

If appropriate, the patient can be further treated with pharmaceutical agents or bioactives that facilitate the survival and function of the transplanted cells. These agents may include, for example, insulin, members of the TGF-β family, including TGF-β1, 2, and 3, bone morphogenic proteins (BMP-2, -3, -4, -5, -6, -7, -11, -12, and -13), fibroblast growth factors-1 and -2, platelet-derived growth factor-AA, and -BB, platelet rich plasma, insulin growth factor (IGF-I, II) growth differentiation factor (GDF-5, -6, -7, -8, -10, -15), vascular endothelial cell-derived growth factor (VEGF), pleiotrophin, endothelin, among others. Other pharmaceutical compounds can include, for example, nicotinamide, glucagon like peptide-I (GLP-1) and II, GLP-1 and 2 mimetibody, Exendin-4, retinoic acid, parathyroid hormone, MAPK inhibitors, such as, for example, compounds disclosed in U.S. Published Application 2004/0209901 and U.S. Published Application 2004/0132729.

The pluripotent stem cells may be differentiated into an insulin-producing cell prior to transplantation into a recipient. In a specific embodiment, the pluripotent stem cells are fully differentiated into β-cells, prior to transplantation into a recipient. Alternatively, the pluripotent stem cells may be transplanted into a recipient in an undifferentiated or partially differentiated state. Further differentiation may take place in the recipient.

Definitive endoderm cells or, alternatively, pancreatic endoderm cells, or, alternatively, β cells, may be implanted as dispersed cells or formed into clusters that may be infused into the hepatic portal vein. Alternatively, cells may be provided in biocompatible degradable polymeric supports, porous non-degradable devices or encapsulated to protect from host immune response. Cells may be implanted into an appropriate site in a recipient. The implantation sites include, for example, the liver, natural pancreas, renal subcapsular space, omentum, peritoneum, subserosal space, intestine, stomach, or a subcutaneous pocket.

To enhance further differentiation, survival or activity of the implanted cells, additional factors, such as growth factors, antioxidants or anti-inflammatory agents, can be administered before, simultaneously with, or after the administration of the cells. In certain embodiments, growth factors are utilized to differentiate the administered cells *in vivo.* These factors can be secreted by endogenous cells and exposed to the administered cells *in situ.* Implanted cells can be induced to differentiate by any combination of endogenous and exogenously administered growth factors known in the art.

The amount of cells used in implantation depends on a number of various factors including the patient's condition and response to the therapy, and can be determined by one skilled in the art.

In one aspect, this disclosure provides a method for treating a patient suffering from, or at risk of developing diabetes. This method involves culturing pluripotent stem cells, differentiating the cultured cells *in vitro* into a β-cell lineage, and incorporating the cells into a three-dimensional support. The cells can be maintained *in vitro* on this support prior to implantation into the patient. Alternatively, the support containing the cells can be directly implanted in the patient without additional *in vitro* culturing. The support can optionally be incorporated with at least one pharmaceutical agent that facilitates the survival and function of the transplanted cells.

Support materials suitable for use for purposes of the present invention include tissue templates, conduits, barriers, and reservoirs useful for tissue repair. In particular, synthetic and natural materials in the form of foams, sponges, gels, hydrogels, textiles, and nonwoven structures, which have been used *in vitro* and *in vivo* to reconstruct or regenerate biological tissue, as well as to deliver chemotactic agents for inducing tissue growth, are suitable for use in practicing the methods of the present invention. See, for example, the materials disclosed in U.S. Patent 5,770,417, U.S. Patent 6,022,743, U.S. Patent 5,567,612, U.S. Patent 5,759,830, U.S. Patent 6,626,950, U.S. Patent 6,534,084, U.S. Patent 6,306,424, U.S. Patent 6,365,149, U.S. Patent 6,599,323, U.S. Patent 6,656,488, U.S. Published Application 2004/0062753 A1, U.S. Patent 4,557,264and U.S. Patent 6,333,029.

To form a support incorporated with a pharmaceutical agent, the pharmaceutical agent can be mixed with the polymer solution prior to forming the support. Alternatively, a pharmaceutical agent could be coated onto a fabricated support, preferably in the presence of a pharmaceutical carrier. The pharmaceutical agent may be present as a liquid, a finely divided solid, or any other appropriate physical form. Alternatively, excipients may be added to the support to alter the release rate of the pharmaceutical agent. In an alternate embodiment, the support is incorporated with at least one pharmaceutical compound that is an anti-inflammatory compound, such as, for example compounds disclosed in U.S. Patent 6,509,369.

The support may be incorporated with at least one pharmaceutical compound that is an anti-apoptotic compound, such as, for example, compounds disclosed in U.S. Patent 6,793,945.

The support may also be incorporated with at least one pharmaceutical compound that is an inhibitor of fibrosis, such as, for example, compounds disclosed in U.S. Patent 6,331,298.

The support may also be incorporated with at least one pharmaceutical compound that is capable of enhancing angiogenesis, such as, for example, compounds disclosed in U.S. Published Application 2004/0220393 and U.S. Published Application 2004/0209901.

The support may also be incorporated with at least one pharmaceutical compound that is an immunosuppressive compound, such as, for example, compounds disclosed in U.S. Published Application 2004/0171623.

The support may also be incorporated with at least one pharmaceutical compound that is a growth factor, such as, for example, members of the TGF-β family, including TGF-β1, 2, and 3, bone morphogenic proteins (BMP-2, -3,-4, -5, -6, -7, -11, -12, and - 13), fibroblast growth factors-1 and -2, platelet-derived growth factor-AA, and -BB, platelet rich plasma, insulin growth factor (IGF-I, II) growth differentiation factor (GDF-5, -6, -8, -10, -15), vascular endothelial cell-derived growth factor (VEGF), pleiotrophin, endothelin, among others. Other pharmaceutical compounds can include, for example, nicotinamide, hypoxia inducible factor 1-alpha, glucagon like peptide-I (GLP-1), GLP-1 and GLP-2 mimetibody, and II, Exendin-4, nodal, noggin, NGF, retinoic acid, parathyroid hormone, tenascin-C, tropoelastin, thrombin-derived peptides, cathelicidins, defensins, laminin, biological peptides containing cell- and heparin-binding domains of adhesive extracellular matrix proteins such as fibronectin and vitronectin, MAPK inhibitors, such as, for example, compounds disclosed in U.S. Published Application 2004/0209901 and U.S. Published Application 2004/0132729.

The incorporation of the cells of the present invention into a scaffold can be achieved by the simple depositing of cells onto the scaffold. Cells can enter into the scaffold by simple diffusion (J. Pediatr. Surg. 23 (1 Pt 2): 3-9 (1988)). Several other approaches have been developed to enhance the efficiency of cell seeding. For example, spinner flasks have been used in seeding of chondrocytes onto polyglycolic acid scaffolds (Biotechnol. Prog. 14(2): 193-202 (1998)). Another approach for seeding cells is the use of centrifugation, which yields minimum stress to the seeded cells and enhances seeding efficiency. For example, Yang et al. developed a cell seeding method (J.Biomed. Mater. Res. 55(3): 379-86 (2001)), referred to as Centrifugational Cell Immobilization (CCI).

The present invention is further illustrated, but not limited by, the following examples.

### REFERENCES

Karvonen, M. et al. Incidence of childhood type 1 diabetes worldwide. Diabetes Mondiale (DiaMond) Project Group. Diabetes Care 23, 1516-26 (2000).
Mathis, D., Vence, L. & Benoist, C. Beta-cell death during progression to diabetes. Nature 414, 792-8 (2001).
Ryan, E.A. et al. Clinical outcomes and insulin secretion after islet transplantation with the Edmonton protocol. Diabetes 50, 710-9 (2001).
Shapiro, A.M. et al. Islet transplantation in seven patients with type 1 diabetes mellitus using a glucocorticoid-free immunosuppressive regimen. N Engl J Med 343, 230-8 (2000).
Cure, P. et al. Improved Metabolic Control and Quality of Life in Seven Patients With Type 1 Diabetes Following Islet After Kidney Transplantation. Transplantation 85, 801-812 (2008).
Fung, M.A. et al. The effect of medical therapy and islet cell transplantation on diabetic nephropathy: an interim report. Transplantation 84, 17-22 (2007).
Brown, L. & Edelman, E.R. Optimal control of blood glucose: the diabetic patient or the machine? Sci Transl Med 2, 27ps18 (2010).
Guo, T. & Hebrok, M. Stem cells to pancreatic beta-cells: new sources for diabetes cell therapy. Endocr Rev 30, 214-27 (2009).
Ricordi, C. & Edlund, H. Toward a renewable source of pancreatic beta-cells. Nat Biotechnol 26, 397-8 (2008).
Rajagopal, J., Anderson, W.J., Kume, S., Martinez, O.I. & Melton, D.A. Insulin staining of ES cell progeny from insulin uptake. Science 299, 363 (2003).
Kroon, E. et al. Pancreatic endoderm derived from human embryonic stem cells generates glucose-responsive insulin-secreting cells in vivo. Nat Biotechnol 26, 443-52 (2008).
D'Amour, K.A. et al. Production of pancreatic hormone-expressing endocrine cells from human embryonic stem cells. Nat Biotechnol 24, 1392-401 (2006).
D'Amour, K.A. et al. Efficient differentiation of human embryonic stem cells to definitive endoderm. Nat Biotechnol 23, 1534-41 (2005).
Matveyenko AV, Georgia S, Bhushan A, Butler PC. Inconsistent formation and non function of insulin positive cells from pancreatic endoderm derived from human embryonic stem cells in athymic nude rats. Am J Physiol Endocrinol Metab. 2010 Jun 29. [Epub ahead of print].
Lee SH, Hao E, Savinov AY, Geron I, Strongin AY, Itkin-Ansari P. Human beta-cell precursors mature into functional insulin-producing cells in an immunoisolation device: implications for diabetes cell therapies. Transplantation. 2009 Apr 15;87(7):983-91.
Yang Z, Chen M, Fialkow LB, Ellett JD, Wu R, Nadler JL. Survival of pancreatic islet xenografts in NOD mice with the theracyte device. Transplant Proc. 2002 Dec;34(8):3349-50.
Panepinto LM and Phillips RW. The Yucatan miniature pig: characterization and utilization in biomedical research. Lab Anim Sci 36: 344-347, 1986.
Larsen MO and Rolin B. Use of the Gottingen minipig as a model of diabetes, with special focus on type 1 diabetes research. Ilar J 45: 303-313, 2004.
Miller ER and Ullrey DE. The pig as a model for human nutrition. Annu Rev Nutr 7: 361-382, 1987
Vodicka P, Smetana K, Jr., Dvorankova B, Emerick T, Xu YZ, Ourednik J, Ourednik V, and Motlik J. The miniature pig as an animal model in biomedical research. Ann N Y Acad Sci 1049: 161-171, 2005.
Kurihara-Bergstrom T, Woodworth M, Feisullin S, and Beall P. Characterization of the Yucatan miniature pig skin and small intestine for pharmaceutical applications. Lab Anim Sci 36: 396-399, 1986.
Swindle MM, Smith AC, Laber-Laird K, and Dungan L. Swine in Biomedical Research: Management and Models. Ilar J 36: 1-5, 1994.
Bellinger DA, Merricks EP, and Nichols TC. Swine models of type 2 diabetes mellitus: insulin resistance, glucose tolerance, and cardiovascular complications. Ilar J 47: 243-258, 2006.
Hainsworth DP, Katz ML, Sanders DA, Sanders DN, Wright EJ, and Sturek M. Retinal capillary basement membrane thickening in a porcine model of diabetes mellitus. Comp Med 52: 523-529, 2002.
Marshall M, Oberhofer H, and Staubesand J. Early micro- and macro-angiopathy in the streptozotocin diabetic minipig. Res Exp Med (Berl) 177: 145-158, 1980.
Phillips RW, Panepinto LM, Will DH, and Case GL. The effects of alloxan diabetes on Yucatan miniature swine and their progeny. Metabolism 29: 40-45, 1980.
Eventov-Friedman S, Tchorsh D, Katchman H, Shezen E, Aronovich A, Hecht G, Dekel B, Rechavi G, Blazar BR, Feine I, Tal O, Freud E, Reisner Y. Embryonic pig pancreatic tissue transplantation for the treatment of diabetes. PLoS Med. 2006 Jul;3(7):e215.
Castaing M, Péault B, Basmaciogullari A, Casal I, Czernichow P, Scharfmann R. Blood glucose normalization upon transplantation of human embryonic pancreas into beta-cell-deficient SCID mice. Diabetologia. 2001 Nov;44(11):2066-76.
Larsen MO, Rolin B, Raun K, Bjerre Knudsen L, Gotfredsen CF, Bock T. Evaluation of beta-cell mass and function in the Gottingen minipig. Diabetes Obes Metab Suppl 2:170-9, 2007
van der Windt DJ, Echeverri GJ, Ijzermans JN, Cooper DK. The choice of anatomical site for islet transplantation. Cell Transplant. 2008;17(9):1005-14.

### Reference EXAMPLE

Cells of the human embryonic stem cell line HI were cultured on MATRIGEL-coated plates (1:30 dilution), and differentiated into pancreatic endocrine precursor cells using the following protocol:
a. RPMI medium (Catalogue#22400, Invitrogen, Ca) supplemented with 2% BSA (Catalog# 152401, MP Biomedical, Ohio), and 100 ng/ml activin A (R&D Systems, MN) plus 20 ng/ml WNT-3a (Catalog# 1324-WN-002, R&D Systems, MN) plus 8 ng/ml of bFGF (Catalog# 100-18B, PeproTech, NJ), for one day followed by treatment with RPMI media supplemented with 2% BSA and 100 ng/ml activin A plus 8 ng/ml of bFGF for an additional two days (Stage 1), then
b. DMEM/F12 (Catalogue#1 1330, Invitrogen, Ca)+ 2% BSA + 50 ng/ml FGF7 for three days (Stage 2), then
c. Different basal media indicated in Table 1 were used, supplemented with 1% B27 (#17504-044, Invitrogen, CA) + 50 ng/ml FGF7 + 0.25 µM Cyclopamine- KAAD (#239804, Calbiochem, CA) + 2 µM Retinoic acid (RA) (Sigma, MO) + 100 ng/ml of Noggin (R & D Systems, MN) for four days (Stage 3), then
d. Different basal media indicated in Table 1 were used, supplemented with 1% B27 (Invitrogen, CA) + 100 ng/ml Noggin + 1 µM ALK5 inhibitor II (Catalog# 616452, Calbiochem, Ca) for three days (Stage 4).

## Claims

1. A population of encapsulated pancreatic endocrine precursor cells for use in a method for lowering blood glucose levels in an animal by transplanting the population of encapsulated pancreatic endocrine precursor cells into the animal, wherein the pancreatic endocrine precursor cells are differentiated *in vitro* from cells expressing markers characteristic of the pancreatic endoderm lineage in a medium supplemented with a factor capable of inhibiting BMP and a TGF-beta receptor I kinase inhibitor and wherein the pancreatic endocrine precursor cells express NKX2.2 and ARX.

2. The population of encapsulated pancreatic endocrine precursor cells for use according to claim 1, wherein the cells are derived from pluripotent stem cells.

3. The population of encapsulated pancreatic endocrine precursor cells for use according to claim 2, wherein the cells are derived from human embryonic stem cells.

4. The population of encapsulated pancreatic endocrine precursor cells for use according to any one of claims 1 to 3, wherein the factor capable of inhibiting BMP is noggin.

5. The population of encapsulated pancreatic endocrine precursor cells for use according to any one of claims 1 to 4, wherein the TGF-beta receptor I kinase inhibitor is ALK5 inhibitor II.

6. The population of encapsulated pancreatic endocrine precursor cells for use according to any one of claims 1 to 5, wherein the medium is DMEM containing 4500mg/l glucose and 1 % B27.

7. The population of encapsulated pancreatic endocrine precursor cells for use according to any one of claims 1 to 6, wherein the cells are cultured in the culture medium for about four days.

8. The population of encapsulated pancreatic endocrine precursor cells for use according to any one of claims 1 to 7, wherein the encapsulated cells are transplanted into the liver, natural pancreas, renal subcapsular space, omentum, peritoneum, subserosal space, intestine, stomach, or a subcutaneous pocket.

9. The population of encapsulated pancreatic endocrine precursor cells for use according to any one of claims 1 to 8, wherein additional factors, such as growth factors, antioxidants or anti-inflammatory agents, are administered before, simultaneously with, or after the transplantation of the population of encapsulated pancreatic endocrine precursor cells.

10. The population of encapsulated pancreatic endocrine precursor cells for use according to any one of claims 1 to 9, wherein a patient suffering from, or at risk of developing, type I or type II diabetes is treated.

## Patentansprüche

1. Population eingekapselter endokriner Pankreas-Vorläuferzellen zur Verwendung in einem Verfahren zur Senkung der Blut-Glucose-Spiegel in einem Tier durch Transplantation der Population eingekapselter endokriner Pankreas-Vorläuferzellen in das Tier, wobei die endokrinen Pankreas-Vorläuferzellen *in vitro* aus Zellen, die Marker, die charakteristisch für die Endoderm-Zelllinie des Pankreas sind, exprimieren, in einem Medium differenzieren, das mit einem Faktor, der in der Lage ist, BMP und einen TGF-beta-Rezeptor-I-Kinase-Inhibitor zu inhibieren, ergänzt wurde und wobei die endokrinen Pankreas-Vorläuferzellen NKX2.2 und ARX exprimieren.

2. Population eingekapselter endokriner Pankreas-Vorläuferzellen zur Verwendung nach Anspruch 1, wobei die Zellen von pluripotenten Stammzellen stammen.

3. Population eingekapselter endokriner Pankreas-Vorläuferzellen zur Verwendung nach Anspruch 2, wobei die Zellen von humanen embryonalen Stammzellen stammen.

4. Population eingekapselter endokriner Pankreas-Vorläuferzellen zur Verwendung nach einem der Ansprüche 1 bis 3, wobei der Faktor, der in der Lage ist, BMP zu inhibieren, Noggin ist.

5. Population eingekapselter endokriner Pankreas-Vorläuferzellen zur Verwendung nach einem der Ansprüche 1 bis 4, wobei der TGF-beta-Rezeptor-I-Kinase-Inhibitor ALK5-Inhibitor II ist.

6. Population eingekapselter endokriner Pankreas-Vorläuferzellen zur Verwendung nach einem der Ansprüche 1 bis 5, wobei das Medium DMEM ist, das 4.500 mg/l Glucose und 1% B27 enthält.

7. Population eingekapselter endokriner Pankreas-Vorläuferzellen zur Verwendung nach einem der Ansprüche 1 bis 6, wobei die Zellen in dem Kulturmedium über etwa vier Tage gezüchtet werden.

8. Population eingekapselter endokriner Pankreas-Vorläuferzellen zur Verwendung nach einem der Ansprüche 1 bis 7, wobei die eingekapselten Zellen in die Leber, den natürlichen Pankreas, den subkapsulären Nierenraum, das Omentum, das Peritoneum, den subserösen Raum, den Darm, den Magen oder eine subkutane Tasche transplantiert werden.

9. Population eingekapselter endokriner Pankreas-Vorläuferzellen zur Verwendung nach einem der Ansprüche 1 bis 8, wobei zusätzliche Faktoren, wie zum Beispiel Wachstumsfaktoren, Antioxidanzien oder anti-inflammatorische Agenzien vor, gleichzeitig mit oder nach der Transplantation der Population eingekapselter endokriner Pankreas-Vorläuferzellen verabreicht werden.

10. Population eingekapselter endokriner Pankreas-Vorläuferzellen zur Verwendung nach einem der Ansprüche 1 bis 9, wobei ein Patient behandelt wird, der an Typ-I- oder Typ-II- Diabetes leidet oder ein Risiko hat, Typ-I- oder Typ-II- Diabetes zu entwickeln.

## Revendications

1. Population de cellules précurseurs endocrines pancréatiques encapsulées pour une utilisation dans une méthode pour abaisser les niveaux sanguins de glucose chez un animal par transplantation dans l'animal de la population de cellules précurseurs endocrines pancréatiques encapsulées, dans laquelle les cellules précurseurs endocrines pancréatiques sont différenciées *in vitro* d'avec des cellules exprimant des marqueurs caractéristiques de la lignée d'endoderme pancréatique dans un milieu complémenté d'un facteur capable d'inhiber la BMP et un inhibiteur de la kinase récepteur I de TGF-bêta, et dans laquelle les cellules précurseurs endocrines pancréatiques expriment NKX2.2 et ARX

2. Population de cellules précurseurs endocrines pancréatiques encapsulées pour une utilisation selon la revendication 1, dans laquelle les cellules dérivent de cellules souches pluripotentes.

3. Population de cellules précurseurs endocrines pancréatiques encapsulées pour une utilisation selon la revendication 2, dans laquelle les cellules dérivent de cellules souches embryonnaires humaines.

4. Population de cellules précurseurs endocrines pancréatiques encapsulées pour une utilisation selon l'une quelconque des revendications 1 à 3, dans laquelle le facteur capable d'inhiber la BMP est la noggine.

5. Population de cellules précurseurs endocrines pancréatiques encapsulées pour une utilisation selon l'une quelconque des revendications 1 à 4, dans laquelle l'inhibiteur de la kinase récepteur I de TGF-bêta est l'inhibiteur d'ALK5 II.

6. Population de cellules précurseurs endocrines pancréatiques encapsulées pour une utilisation selon l'une quelconque des revendications 1 à 5, dans laquelle le milieu est du DMEM contenant 4500 mg/l de glucose et 1 % de B27.

7. Population de cellules précurseurs endocrines pancréatiques encapsulées pour une utilisation selon l'une quelconque des revendications 1 à 6, dans laquelle les cellules sont cultivées dans le milieu de culture pendant environ quatre jours.

8. Population de cellules précurseurs endocrines pancréatiques encapsulées pour une utilisation selon l'une quelconque des revendications 1 à 7, dans laquelle les cellules encapsulées sont transplantées dans le foie, le pancréas naturel, l'espace sous-capsulaire rénal, l'épiploon, le péritoine, l'espace sous-séreux, l'intestin, l'estomac, ou une poche sous-cutanée.

9. Population de cellules précurseurs endocrines pancréatiques encapsulées pour une utilisation selon l'une quelconque des revendications 1 à 8, dans laquelle des facteurs additionnels, tels que des facteurs de croissance, des antioxydants ou des agents anti-inflammatoires, sont administrés avant, en même temps que, ou après, la transplantation de la population de cellules précurseurs endocrines pancréatiques encapsulées.

10. Population de cellules précurseurs endocrines pancréatiques encapsulées pour une utilisation selon l'une quelconque des revendications 1 à 9, dans laquelle un patient souffrant de, ou risquant de développer, un diabète de type I ou de type II, est traité.
